# EUROPEAN PATENT APPLICATION

(11) **EP 1 949 854 A1**
(43) Date of publication of application: **30.07.2008**
(21) Application number: 06832417.7
(22) Date of filing: 02.11.2006
(51) Int. Cl.: A61B 5/022

(54) **ELECTRONIC SPHYGMOMANOMETER AND DATA DISPLAY METHOD IN ELECTRONIC SPHYGMOMANOMETER**

(30) Priority: 08.11.2005 JP 2005323705
(71) Applicant: Omron Healthcare Co., Ltd., Kyoto 615-0084 (JP)
(72) Inventor: KISHIMOTO, Hiroshi, c/o OMRON HEALTHCARE CO., LTD., Kyoto-shi Kyoto 615-0084 (JP); SAWANOI, Yukiya, c/o OMRON HEALTHCARE CO., LTD., Kyoto-shi Kyoto 615-0084 (JP)
(74) Representative: Wilhelms · Kilian & Partner Patentanwälte
(86) International application number: PCT/JP2006/321951
(87) International publication number: WO 2007/055148

(57) **Abstract**

A memory (13) of an electronic blood pressure monitor stores blood pressure data (SBP, DBP) measured in the past in association with measurement time data (T). Each time a subject operates a month update switch (23), representative blood pressure data of each month is read from the memory and displayed. Each time a day update switch (24) is operated, representative blood pressure data of each day is read and displayed. Each time an individual switch (25) is operated, blood pressure data is read and displayed in the order of measurement time in a day.

## Description

### TECHNICAL FIELD

The present invention relates to an electronic blood pressure monitor and a data display method in the electronic blood pressure monitor, and more particularly to an electronic blood pressure monitor retrieving measurement result data obtained as a result of measurement at a desired time out of a large amount of data of blood pressure measurement results that have been stored and displaying the retrieved data, and a data display method in the electronic blood pressure monitor.

### BACKGROUND ART

Lifestyle-related diseases due to high blood pressure have been common in recent years, and a blood pressure value is used as an index for health care and management. Blood pressure management is important, and measurement of blood pressure is conducted for that purpose. The conventional techniques are as follows.

Registered Japanese Utility Model No. 3020497 shows a digital automatic-storage-type blood pressure monitor that includes a recall button of a push-button control circuit and is capable of recalling data of previous several measurements of a subject. In addition, Japanese Patent Laying-Open No. 4-221528 shows an apparatus having a function to identify a blood pressure value, identification data, and a time and day of measurement and to select and display only a blood pressure value provided with the same identification data, as well as a function to display a trend graph in accordance with a measurement status.
Patent Document 1: Registered Japanese Utility Model No. 3 020497
Patent Document 2: Japanese Patent Laying-Open No. 4-221528

### DISCLOSURE OF THE INVENTION

### PROBLEMS TO BE SOLVED BY THE INVENTION

According to the configurations disclosed in Registered Japanese Utility Model No. 3020497 and Japanese Patent Laying-Open No. 4-221528 above, a memory of a large capacity is provided to store a large amount of measurement data therein, so that data sufficient for blood pressure management can be prepared in the memory. In order to read measurement data on a specific date from the memory and to display the read data, a call button should be pressed. When the call button is pressed, the measurement data and the date data indicating the date of measurement are read from the memory and displayed on a display unit. When a subject determines that the displayed measurement data does not indicate the specific date, the subject presses the call button. Each time the call button is pressed, next measurement data and date data are read from the memory and displayed.

Thus, conventionally, in order to call measurement data on a specific date from the memory, it has been necessary to repeatedly press the call button and check the displayed data until the measurement data on the specific date is called, and operability for calling the measurement data has not been satisfactory.

Therefore, an object of the present invention is to provide an electronic blood pressure monitor capable of readily reading data measured at a desired measurement time from a memory and displaying the data, and a data display method in the electronic blood pressure monitor.

### MEANS FOR SOLVING THE PROBLEMS

According to one aspect of the present invention, an electronic blood pressure monitor includes: a blood pressure measurement unit having a cuff attached to a blood pressure measurement site, a pressurization and depressurization unit adjusting a pressure to be applied to the cuff, a pressure detection unit detecting a pressure in the cuff adjusted by the pressurization and depressurization unit, and a blood pressure calculation unit calculating a blood pressure based on the pressure detected by the pressure detection unit; a memory; a display unit; a storing unit storing in the memory, data of the blood pressure calculated by the blood pressure calculation unit in association with measurement time data indicating a time when blood pressure of the blood pressure data was measured; a storage operation unit operated for designation of a desired time; a reading unit retrieving and reading the blood pressure data associated with the measurement time data indicating the desired time from the memory, based on operation detail of the storage operation unit; and a display control unit causing the display unit to display the blood pressure data read by the reading unit.

Therefore, according to the electronic blood pressure monitor, when a subject operates the storage operation unit for designating a desired time, the reading unit retrieves and reads from the memory, the blood pressure data associated with the measurement time data indicating the desired time, based on the operation detail, and the blood pressure data is displayed through the display control unit. Therefore, the subject can cause blood pressure data measured at a desired time, out of a large amount of blood pressure data stored in the memory, to selectively be read and displayed simply by performing an operation to designate a desired time, without repeating a series of procedures including operation for reading, data read, and display many times.

Preferably, the measurement time data includes data indicating a month when measurement was conducted, and the storage operation unit includes a month operation unit for successively retrieving and reading representative blood pressure data out of one or more piece of the blood pressure data associated with the measurement time data indicating each month, each time it is operated.

Therefore, each time the subject performs the operation, the representative blood pressure data of each month can be read and displayed.

Preferably, the measurement time data includes data indicating a month and a day when measurement was conducted, and the storage operation unit includes a day operation unit for successively retrieving and reading representative blood pressure data out of one or more piece of the blood pressure data associated with the measurement time data indicating each day in a prescribed month, each time it is operated.

Therefore, each time the subject performs the operation, the representative blood pressure data of each day in a prescribed month can be read and displayed.

Preferably, the storage operation unit includes an individual operation unit for successively retrieving and reading one or more piece of the blood pressure data associated with the measurement time data indicating prescribed month and day, each time it is operated.

Therefore, each time the subject performs the operation, the blood pressure data measured and stored on a prescribed day in a prescribed month can successively be read from the memory and displayed.

Preferably, the measurement time data includes data indicating a month, a day, and hour and minute when measurement was conducted, and each time the storage operation unit is operated, the storage operation unit selectively sets an operating mode of the reading unit to any of a month operation mode for retrieving and reading representative blood pressure data out of one or more piece of the blood pressure data associated with the measurement time data indicating each month, a day operation mode for successively retrieving and reading representative blood pressure data out of one or more piece of the blood pressure data associated with the measurement time data indicating each day in a prescribed month each time it is operated, and an individual operation mode for retrieving and reading one or more piece of the blood pressure data associated with the measurement time data indicating prescribed month and day each time it is operated.

Therefore, each time the subject operates the storage operation unit, the operating mode of the reading unit for reading the blood pressure data from the memory can selectively be switched to any of the month operation mode, the day operation mode and the individual operation mode described above. Therefore, the blood pressure data associated with the desired measurement time can quickly be read from the memory and displayed.

Preferably, the storage operation unit includes a calendar display unit displaying a calendar on a screen for designation of the desired measurement time, and retrieves and reads representative blood pressure data out of one or more piece of the blood pressure data associated with the measurement time data indicating a month designated in the calendar displayed on the calendar display unit.

Preferably, representative blood pressure data out of one or more piece of the blood pressure data associated with the measurement time data indicating a month and a day designated in the calendar displayed on the calendar display unit is retrieved and read.

Therefore, as the desired time can be designated on the displayed calendar, the storage operation is facilitated.

Preferably, as to the representative blood pressure data, the associated measurement time data indicates a time closest to a current time kept by the timer. Therefore, if there are a plurality of pieces of blood pressure data associated with time data indicating the desired time, blood pressure data measured most recently among those is read and displayed.

### EFFECTS OF THE INVENTION

According to the present invention, the subject can cause the blood pressure data measured at the desired time, out of a large amount of blood pressure data stored in the memory, to selectively be read and displayed simply by performing an operation to designate a desired time, without repeating a series of procedures including operation for reading, data read and display many times.

### BRIEF DESCRIPTION OF THE DRAWINGS

Fig. 1 is a diagram of a hardware configuration of an electronic blood pressure monitor according to each embodiment.
Fig. 2 is a diagram showing the overall electronic blood pressure monitor according to a first embodiment.
Fig. 3 illustrates an example of storage of measurement data in the electronic blood pressure monitor according to each embodiment.
Fig. 4 is a diagram of a functional configuration of the electronic blood pressure monitor according to the first embodiment.
Fig. 5 is a flowchart showing a procedure for measuring blood pressure according to each embodiment.
Fig. 6 illustrates an example of display according to the first embodiment.
Fig. 7 is a diagram showing an overall electronic blood pressure monitor according to a second embodiment.
Fig. 8 is a diagram of a functional configuration of the electronic blood pressure monitor according to the second embodiment.
Fig. 9 illustrates an example of display according to the second embodiment.
Fig. 10 is a diagram showing an overall electronic blood pressure monitor according to a third embodiment.
Fig. 11 is a diagram of a functional configuration of the electronic blood pressure monitor according to the third embodiment.

### DESCRIPTION OF THE REFERENCE SIGNS

1 electronic blood pressure monitor; 2 display unit; 4 operation unit; 13 memory; 16 CPU; 201 blood pressure calculation unit; 211 memory storing unit; 221, 222, 223 storage operation unit; 231 memory read unit; 241 display control unit; 251 time keeping data; 252 operation signal; and F1 flag.

### BEST MODES FOR CARRYING OUT THE INVENTION

Each embodiment of the present invention will be described hereinafter in detail with reference to the drawings. In the description below, the same elements and components have the same reference characters allotted. Their label and function are also identical. Therefore, detailed description thereof will not be repeated.

### (First Embodiment)

### (Configuration)

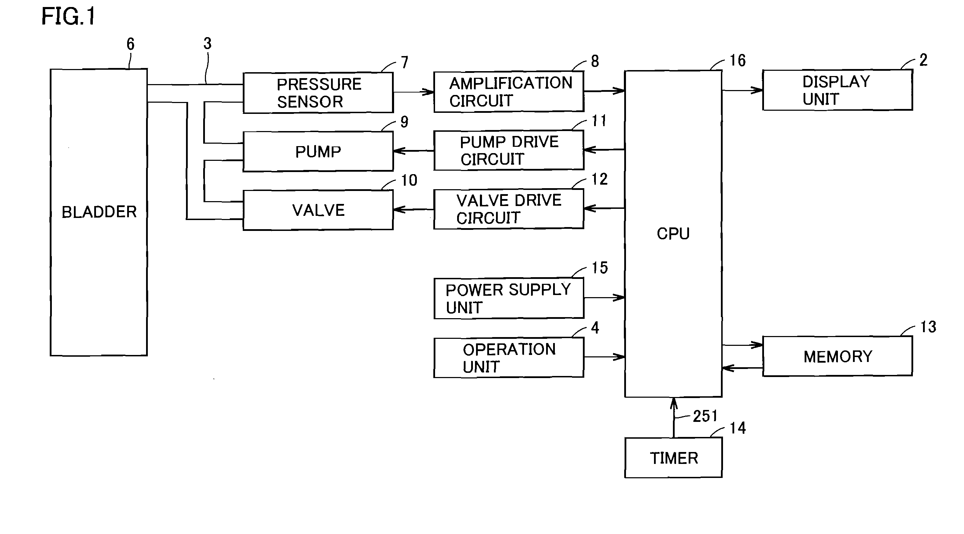

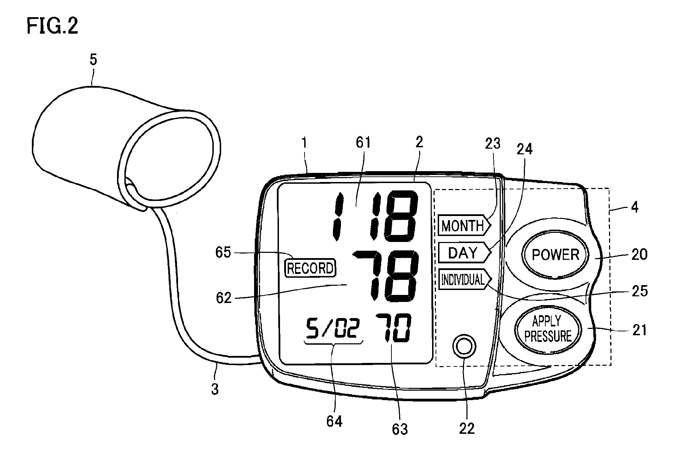

A hardware configuration of an electronic blood pressure monitor applied to each embodiment is shown in Fig. 1. Fig. 2 shows an overview of an electronic blood pressure monitor 1 in a first embodiment.

Referring to Fig. 2, electronic blood pressure monitor 1 according to the present embodiment includes a cuff 5 attached to a blood pressure measurement site of a subject, for application of a pressure by means of air pressure, and an air tube 3 connecting a main body of the blood pressure monitor and cuff 5 to each other. The main body of the blood pressure monitor includes a display unit 2 provided to allow the subject to check display content and an operation unit 4 provided to permit external operation by the subject.

Operation unit 4 includes a power switch 20, a measurement switch 21 instructing start of pressure application, that is, start of measurement, a clock setting switch 22, and a month update switch 23, a day update switch 24 and an individual switch 25 operated for calling stored data. Power switch 20 is operated in order to turn on/off power of the blood pressure monitor main body. Measurement switch 21 is operated in order to instruct start or stop of measurement of blood pressure. Month update switch 23, day update switch 24 and individual switch 25 will be described later.

Referring to Fig. 1, electronic blood pressure monitor 1 includes a pressure sensor 7 for outputting variation in a pulse pressure at a measurement site detected via bladder 6 contained in cuff 5 as a pulse wave signal, an amplification circuit 8 amplifying a voltage signal indicating the pressure output from pressure sensor 7, a pump 9 and a valve 10 for adjusting a pressure application (air pressure) level of bladder 6, a pump drive circuit 11 driving pump 9, a valve drive circuit 12 for adjusting opening and closing of valve 10, display unit 2, a memory 13, operation unit 4, a timer 14 operating to keep time and outputting time keeping data 251, a power supply unit 15, and a CPU (Central Processing Unit) 16 controlling each of these units. A user can set time keeping data 251 of timer 14 by operating clock setting switch 22.

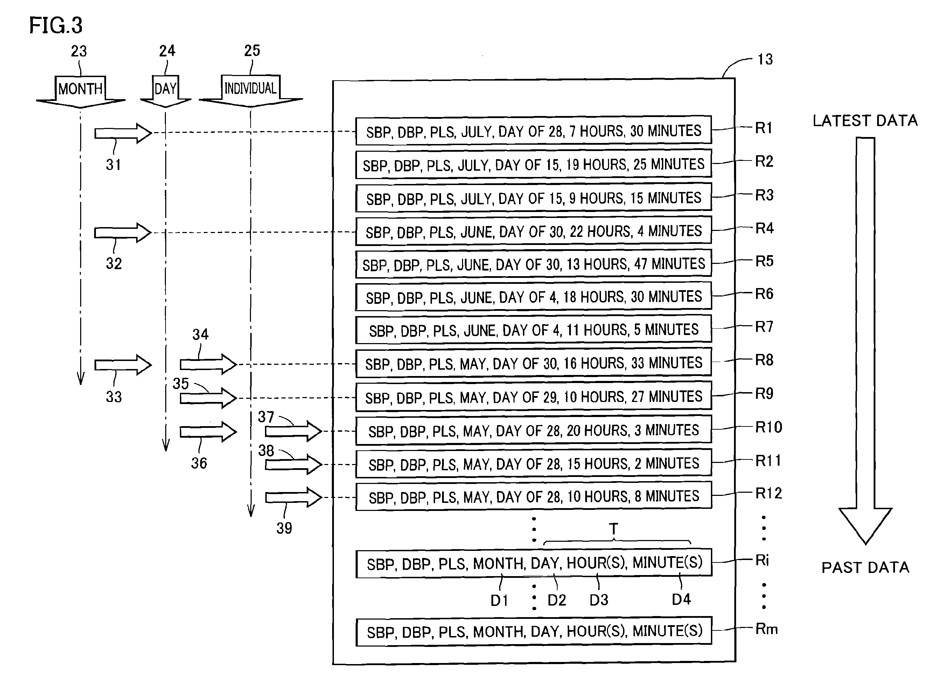

Referring to Fig. 3, measurement result data is stored in memory 13 for each blood pressure measurement in the order of measurement time, in a unit of record Ri (i=1, 2, 3, ... m). Record Ri includes data SBP indicating a systolic blood pressure, data DBP indicating a diastolic blood pressure, data PLS indicating a pulse rate, and time data T indicating a measurement time. Time data T is constituted of data D1 indicating a month, data D2 indicating a day, data D3 indicating hour(s), and data D4 indicating minute(s) when measurement was conducted. The data should only be stored in memory 13 in association with each other each time the blood pressure is measured, without limited to a storage manner using record Ri. It is noted that time data T is obtained based on time keeping data 251 output by timer 14.

### (Functional Configuration)

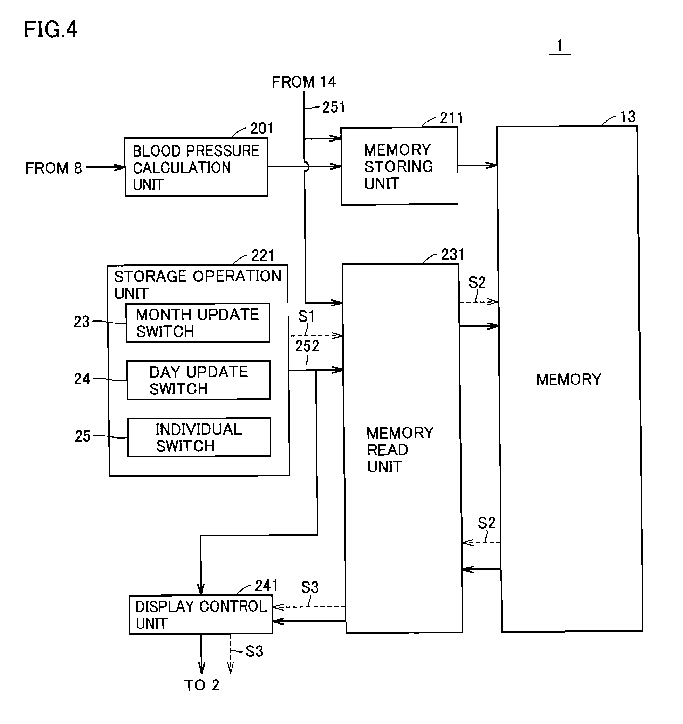

Fig. 4 shows a functional configuration of electronic blood pressure monitor 1 according to the first embodiment. Electronic blood pressure monitor 1 includes a blood pressure calculation unit 201 for calculating a systolic blood pressure, a diastolic blood pressure and a pulse rate based on a voltage signal provided from amplification circuit 8, a memory storing unit 211 receiving input of data (data SBP, data DBP and data PLS) calculated by blood pressure calculation unit 201 and time keeping data 251 provided from timer 14, generating record Ri based on the input data, and storing generated record Ri in memory 13, a storage operation unit 221 operated in order to call data in memory 13 and outputting an operation signal 252 indicating the operation detail, a memory read unit 231 reading data from memory 13 based on provided operation signal 252 and time keeping data 251, and a display control unit 241 for causing display unit 2 to display data based on data read by memory read unit 231 and operation signal 252 provided from storage operation unit 221. CPU 16 functions as each unit except for memory 13 in Fig. 4. Namely, a function of each unit is implemented in such a manner that CPU 16 reads and executes a program corresponding to each unit that is stored in advance in a prescribed area of memory 13. It is noted that each unit in Fig. 4 may be prepared as a circuit.

In Fig. 4, an arrow in solid line indicates a flow of data or a signal, and an arrow in dotted line schematically indicates steps S1 to S3 configuring a procedure for calling blood pressure measurement data which will be described later.

Though storage operation unit 221 is shown to have month update switch 23, day update switch 24 and individual switch 25 in Fig. 4, this means that, when the subject operates any of month update switch 23, day update switch 24 and individual switch 25, storage operation unit 221 outputs operation signal 252 corresponding to the operated switch.

When the user operates any of month update switch 23, day update switch 24 and individual switch 25, storage operation unit 221 provides operation signal 252 indicating which switch was operated to memory read unit 231 and display control unit 241. Memory read unit 231 retrieves record Ri in memory 13 based on provided operation signal 252 and time keeping data 251 provided from timer 14, and outputs resultant record Ri to display control unit 241. Display control unit 241 causes display unit 2 to display read record Ri based on provided operation signal 252.

### (Measurement Procedure)

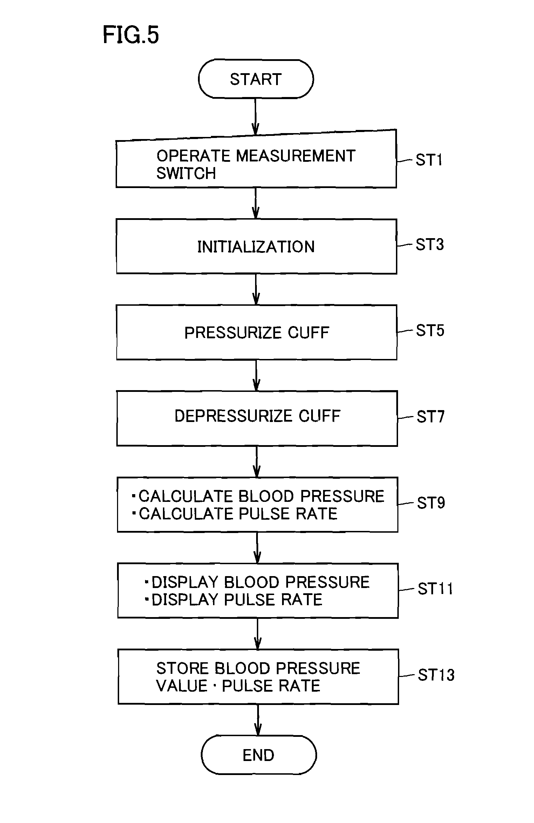

A blood pressure measurement operation using electronic blood pressure monitor 1 will be described with reference to the flowchart in Fig. 5. The program in accordance with the flowchart in Fig. 5 is stored in advance in a prescribed area in memory 13, and the program is read from memory 13 and executed by CPU 16. It is assumed here that, in response to an operation of power switch 20 of electronic blood pressure monitor 1, CPU 16 controls power supply unit 15 so as to supply power to each unit.

Initially, as the subject places cuff 5 around a measurement site and operates measurement switch 21 (step ST (hereinafter simply abbreviated as ST) 1), CPU 16 controls each unit to exhaust air in bladder 6 and to carry out 0mmHg correction of pressure sensor 7 as processing for initializing electronic blood pressure monitor 1 (ST3).

When the initialization processing ends, CPU 16 controls each unit to increase the pressure in bladder 6 to a level as high as the systolic blood pressure+40mmHg of the subject (ST5), and thereafter gradually reduce the pressure in bladder 6 (ST7). In the process of pressure reduction, the pressure in bladder 6 is detected by pressure sensor 7 and amplification circuit 8. Blood pressure calculation unit 201 calculates blood pressure (systolic blood pressure and diastolic blood pressure) values and a pulse rate based on a voltage signal output from amplification circuit 8, that are temporarily stored in a not-shown internal memory of CPU 16 (ST9). CPU 16 reads the data of the blood pressure values and the pulse rate calculated by blood pressure calculation unit 201 from the internal memory and provides the data to display control unit 241. Display control unit 241 causes display unit 2 to display the provided data (ST11). The processing for pressure application and reduction for blood pressure measurement and the procedure for calculating the blood pressure and the pulse rate performed by blood pressure calculation unit 201 are the same as those of the electronic blood pressure monitor that has conventionally been known. Though the blood pressure is measured herein during the course of pressure reduction, the blood pressure may be measured during the course of pressure application.

When calculation and display of the blood pressure and the pulse ends, memory storing unit 211 obtains time data T based on provided time keeping data 251, generates record Ri constituted of obtained time data T and data SBP, DBP and PLS indicating the blood pressure (systolic blood pressure and diastolic blood pressure) values and the pulse rate read from the internal memory of CPU 16, and stores generated record Ri in memory 13 (ST13). A series of blood pressure measurement processes thus ends.

In the procedure in Fig. 5, though time data T indicates the time when the blood pressure measurement ends as the time of blood pressure measurement, it may indicate the time when the blood pressure measurement is started.

### (Procedure for Calling Blood Pressure Measurement Data)

The blood pressure measurement data (record Ri) stored in memory 13 in Fig. 3 is called in accordance with an external operation. Here, "call" refers to an operation to read data from memory 13 and display the read data on display unit 2. A calling operation by memory read unit 231 based on operation signal 252 provided from storage operation unit 221 will be described. It is assumed here that time keeping data 251 indicates July 29 and no data is displayed on display unit 2.

Thus, display unit 2 displays measurement data at the time of blood pressure measurement in accordance with the procedure in Fig. 5, and also displays the data measured and recorded in the past after it is read from memory 13, as follows. In display in the latter case, an icon 65 is displayed as shown in Fig. 2 and the subject is notified that the data being displayed is the past measurement data that has been recorded.

Referring to Fig. 4, the procedure for calling the blood pressure measurement data includes step S 1 performed by storage operation unit 221 accepting an operation for designating a desired time and outputting operation signal 252, reading step S2 performed by memory read unit 231 retrieving and reading from memory 13, the blood pressure measurement data associated with time data T indicating the desired time based on operation signal 252 output in step S 1, and display step S3 performed by display control unit 241 causing display unit 2 to display the blood pressure measurement data read in step S2. Specifically, the procedure is as follows.

Initially, when month update switch 23 is operated, memory read unit 231 searches memory 13 based on operation signal 252 shown with an arrow 31 and reads record Ri. Specifically, out of one or more record Ri storing time data T including data D1 indicating the most recent month (the current month or the most recent month although not current), record Ri storing time data T having data D2 to D4 indicating the most recent time is retrieved and read, and read record Ri is provided to display control unit 241. For example, in Fig. 3, among records R1 to R3 of which data D1 indicates "July", record R1 indicating the most recent time (the time in the past that is closest to the current time) is retrieved and read, and displayed. Here, memory read unit 231 determines whether the time indicated by time data T is the same as the current time indicated by time keeping data 251, or whether it indicates the most recent time (time in the past) by comparing provided time keeping data 251 with time data T of each of records R1 to R3, and reads record R1 based on the result of determination.

Then, when month update switch 23 is operated, out of one or more record Ri storing time data T including data D1 indicating a month most recent next to the month indicated by data D1 of record R1 displayed on display unit 2, memory read unit 231 retrieves and reads record Ri storing time data T including data D2 to D4 that indicates the most recent time based on operation signal 252 shown with an arrow 32, and provides read record Ri to display control unit 241. In Fig. 3, among records R4 to R7 indicative of "June" which is a month most recent next to "July" indicated by data D1 of record R1 displayed on display unit 2, record R4 of which time data T indicates the most recent time is read and displayed. Here, "a month most recent next to the month indicated by data D1" refers to a most recent month among the months in the past that precede the month indicated by data D1.

Thus, each time month update switch 23 is operated, record Ri displayed on display unit 2 goes back to past record Ri by the month. Then, out of one or more record Ri having data D1 indicating the month, record Ri having time data T indicating the most recent time is selectively read and displayed for each retroactive month.

For example, it is assumed that day update switch 24 is operated while record R8 of "May" is retrieved and displayed on display unit 2 as a result of operation of month update switch 23 and resultant operation signal 252 shown with an arrow 33. Here, based on operation signal 252 issued by day update switch 24 shown with an arrow 34, memory read unit 231 specifies one or more record Ri having data D2 indicating the most recent day out of one or more record Ri storing data D1 indicative of "May" indicated by record Ri that is being displayed. Then, out of specified record Ri, memory read unit 231 searches memory 13 for record Ri having data D3 and D4 indicating the latest hour(s) and minute(s), and provides read record Ri to display control unit 241. In Fig. 3, out of records R8 to R12 of which data D1 indicates "May", record R8 of which time data T indicates the most recent time is read and displayed.

Thereafter, when day update switch 24 is operated, based on operation signal 252 shown with an arrow 35, out of one or more record R8 to R12 in memory 13 storing data D1 indicative of "May" indicated by record R8 that is being displayed on display unit 2, memory read unit 231 specifies one or more record Ri storing data D2 indicating a day most recent next to the day indicated by data D2 in record R8 that is being displayed. Then, out of specified record Ri, record Ri storing time data T indicating the most recent time is retrieved and read from memory 13, and read record Ri is provided to display control unit 241. In Fig. 3, record R9 is read and displayed.

Thereafter, when day update switch 24 is operated, based on operation signal 252 shown with an arrow 36, out of one or more record R8 to R12 storing data D1 indicative of "May" indicated by record R9 that is being displayed on display unit 2, memory read unit 231 specifies one or more record R10 to R12 storing data D2 indicating a day most recent next to the day indicated by data D2 in record R9. Then, out of specified records R10 to R12, record Ri storing time data T indicating the most recent time is retrieved and read from memory 13, and read record Ri is provided to display control unit 241. In Fig. 3, record R10 is read and displayed. Here, the "day most recent next to the day indicated by data D2" refers to a most recent day among the days in the past that precede the day indicated by data D2.

Thus, each time day update switch 24 is operated, one or more record Ri having data D1 indicative of the month the same as that indicated by record Ri displayed on display unit 2 goes back to past record Ri by the day. Then, out of one or more record Ri indicating the retroactive day, record Ri indicating most recent time data T is selectively read and displayed.

For example, it is assumed that individual switch 25 is operated while record R10 indicative of "May 28" is retrieved and displayed on display unit 2 as a result of operation of day update switch 24 and resultant operation signal 252 shown with arrow 36. Here, based on operation signal 252 issued by individual switch 25 shown with an arrow 37, memory read unit 231 retrieves and reads from memory 13 record Ri storing time data T indicating the most recent time out of one or more record Ri having data D 1 and D2 indicative of "May 28" indicated by record Ri that is being displayed, and provides read record Ri to display control unit 241. In Fig. 3, among records R10 to R12 having data D1 and D2 indicative of "May 28" the same as indicated by record R10 that is being displayed, record R10 having data D3 and D4 indicating the most recent time is selectively read from memory 13 and displayed.

Thereafter, when individual switch 25 is operated, based on operation signal 252 shown with an arrow 38, memory read unit 231 retrieves and reads record Ri storing time data T indicating the most recent hour(s) and minute(s) next to the time indicated by data D3 and D4 in time data T of record R10 that is being displayed and provides read record Ri to display control unit 241. In Fig. 3, record R11 is read. Here, the "most recent hour(s) and minute(s) next to the time indicated by data D3 and D4" refers to a most recent time (hour(s) and minute(s)) among the times in the past that precede the time expressed as hour(s) and minute(s) indicated by data D3 and D4.

When individual switch 25 is further operated while record R11 is displayed, based on operation signal 252 shown with an arrow 39, memory read unit 231 retrieves and reads record Ri having data D2 and D3 indicating the most recent hour(s) and minute(s) next to the hour(s) and minute(s) indicated by data D2 and D3 of record R11 that is being displayed, out of records R10 to R12 having data D1 and D2 indicative of "May 28" the same as indicated by record R10 that is being displayed, and provides read record Ri to display control unit 241. In Fig. 3, record R12 is read and displayed.

Thus, each time individual switch 25 is operated, within a range of one or more record Ri having data D 1 and D2 indicating the month and the day the same as indicated by record Ri displayed on display unit 2 among records Ri stored in memory 13, the record goes back to record Ri in the past by the hour and the minute, and the past record can selectively be read from memory 13 and displayed.

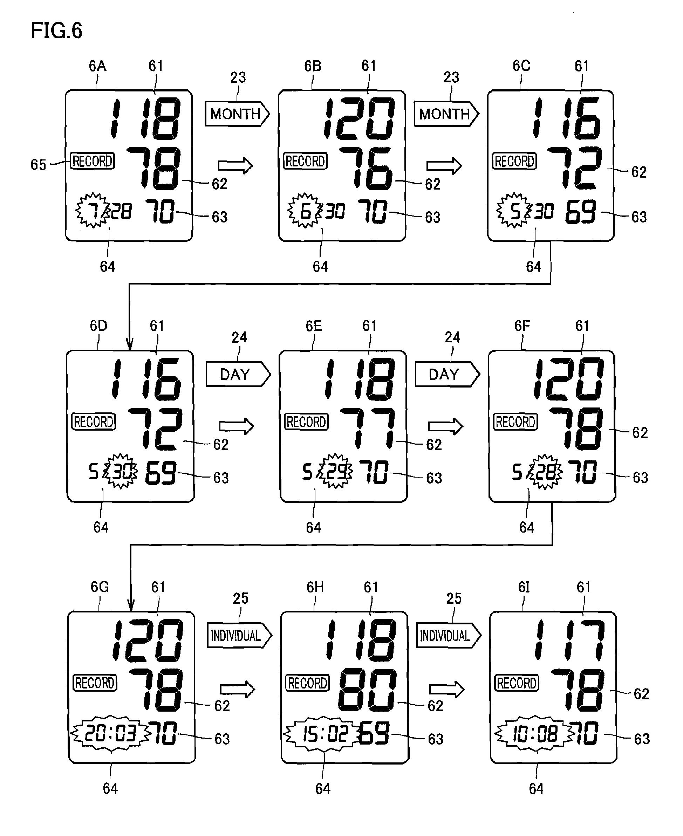

Fig. 6 shows display examples 6A to 6I on display unit 2 in response to calling of blood pressure measurement data (record Ri) stored in memory 13 in the first embodiment. In display examples 6A to 6I, data SBP, data DBP, data PLS, and time data T of record Ri are displayed on display unit 2 after they are converted to data 61 to 64 for display by display control unit 241.

Initially, month update switch 23 is operated, and record R1 is read from memory 13 and displayed through display control unit 241 as in example 6A. As operation signal 252 is provided also to display control unit 241 in parallel to memory read unit 231, display control unit 241 allows display of record Ri read from memory 13 based on provided operation signal 252. Here, as to data 64 corresponding to time data T, a display manner of a value indicated by data D 1 is made different from a display manner of other data based on the fact that operation signal 252 indicates that month update switch 23 has been operated. For example, the value blinks.

Thereafter, each time month update switch 23 is operated, records R1→R4→R8 are successively read from memory 13, and display on display unit 2 changes from example 6A → example 6B → example 6C. In example 6B and example 6C as well, as in example 6A, the display manner of the value indicated by data D1 of data 64 is different from the display manner of other data.

Therefore, by making the display manner of the value (value indicating the "month") corresponding to data D1 of data 64 different from the display manner of others, the subject can be notified that, among the data measured in the month indicated by data 64, the blood pressure data and the pulse rate data obtained as a result of most recent measurement (on the last occasion in that month) are displayed.

When day update switch 24 is operated while example 6C is displayed on display unit 2, the screen on display unit 2 changes from example 6C to example 6D based on record R8. Display control unit 241 makes the display manner of a value indicated by data D2 on display unit 2 different from the display manner of other data based on the fact that provided operation signal 252 indicates that day update switch 24 has been operated. For example, the value blinks.

Thereafter, each time day update switch 24 is operated, records R8→R9→R10 are successively read from memory 13, and display on display unit 2 changes from example 6D → example 6E → example 6F. In example 6E and example 6F as well, as in example 6D, the display manner of the value indicated by data D2 of data 64 is different from the display manner of other data.

Therefore, by making the display manner of the value (value indicating a "day") corresponding to data D2 of data 64 different from the display manner of others, the subject can be notified that, among the data measured on the date indicated by data 64, the blood pressure data and the pulse rate data measured most recently (last measurement data among data measured on that date) are displayed.

When individual switch 25 is operated while example 6F is displayed, the screen on display unit 2 changes from example 6F to example 6G based on record R10. Display control unit 241 makes the display manner of values indicated by data D3 and D4 of data 64 on display unit 2 different from the display manner of other data based on the fact that provided operation signal 252 indicates that individual switch 25 has been operated. For example, the values blink.

Thereafter, each time individual switch 25 is operated, records R10→R11→R12 are successively read from memory 13, and display on display unit 2 changes from example 6G → example 6H → example 6I. In example 6H and example 6I as well, as in example 6G, the display manner of the values indicated by data D3 and D4 of data 64 is different from the display manner of other data.

Therefore, by making the display manner of the values (values indicating the "hour(s) and minute(s)") corresponding to data D3 and D4 of data 64 different from the display manner of others, the subject can be notified that the blood pressure data and the pulse rate data different in the time of measurement in one day are displayed.

Thus, by providing month update switch 23, day update switch 24 and individual switch 25 as switches for calling the blood pressure measurement data stored in memory 13, measurement data on a desired date can readily be called. Namely, instead of retrieving and displaying measurement data stored in memory 13 simply in the descending order from latest data to past data as in a conventional example, representative measurement data of each month can be called from memory 13 for each operation of month update switch 23 and representative measurement data of each day (latest and most recent) can be called from memory 13 for each operation of day update switch 24. Consequently, the data at a desired measurement time out of a large amount of measurement data stored in memory 13 can readily be called.

### (Second Embodiment)

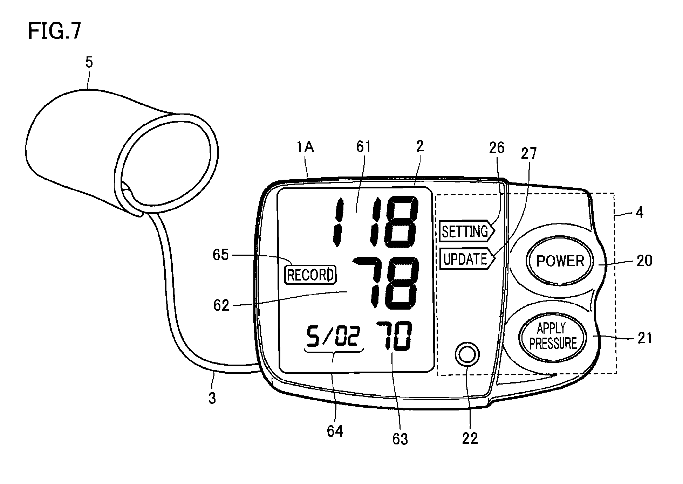

In a second embodiment, an electronic blood pressure monitor 1A shown in Fig. 7 is employed. Electronic blood pressure monitor 1A in Fig. 7 is different from electronic blood pressure monitor 1 in Fig. 2 in that a setting switch 26 and an update switch 27 are provided instead of month update switch 23, day update switch 24 and individual switch 25 in Fig. 2 and that a flag F1 is stored in an internal memory (not shown) of CPU 16. A value of flag F1 is updated by CPU 16. As other configurations are the same as in Fig. 2, description thereof will not be provided.

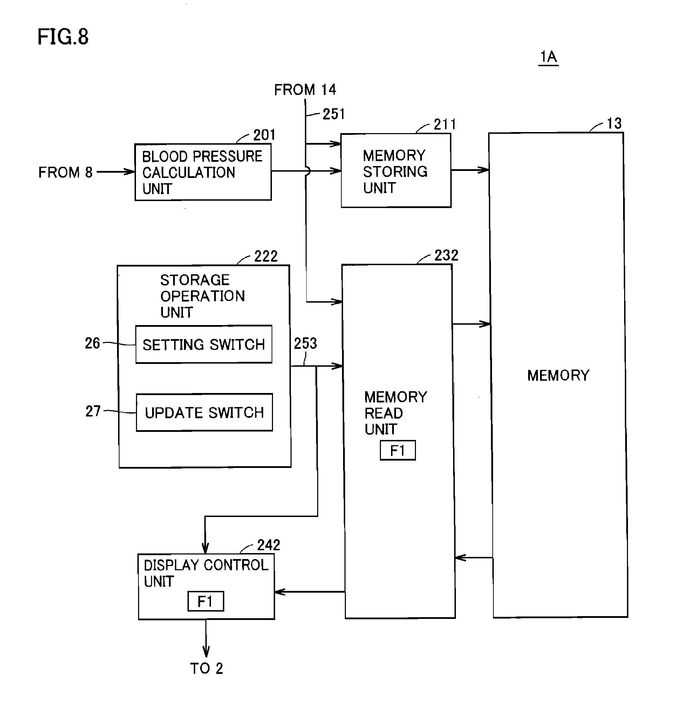

Fig. 8 shows a functional configuration of electronic blood pressure monitor 1A according to the present second embodiment. The functional configuration in Fig. 8 is different from the functional configuration in Fig. 4 in the first embodiment in that a storage operation unit 222, a memory read unit 232, and a display control unit 242 are provided instead of storage operation unit 221, memory read unit 231, and display control unit 241 in Fig. 4, respectively. The functional configuration is otherwise the same as in Fig. 4. Storage operation unit 222 outputs an operation signal 253 in accordance with an operation of setting switch 26 and update switch 27.

Flag F1 is stored in the internal memory (not shown) of CPU 16 and referred to by memory read unit 232 and display control unit 242. Flag F1 instructs an operating mode of memory read unit 232 and display control unit 242. Fig. 8 shows that flag F1 is stored in memory read unit 232 and display control unit 242 for the sake of illustration. Even when electronic blood pressure monitor 1A is turned off, the value of flag F1 is held. Each time setting switch 26 is operated, in response to operation signal 253, flag F 1 is successively rewritten to a value setting the operating mode of memory read unit 232 and display control unit 242 to a mode of reading the measurement data by the month, a mode of reading the measurement data by the day, and a mode of reading measurement data by the hour(s) and minute(s).

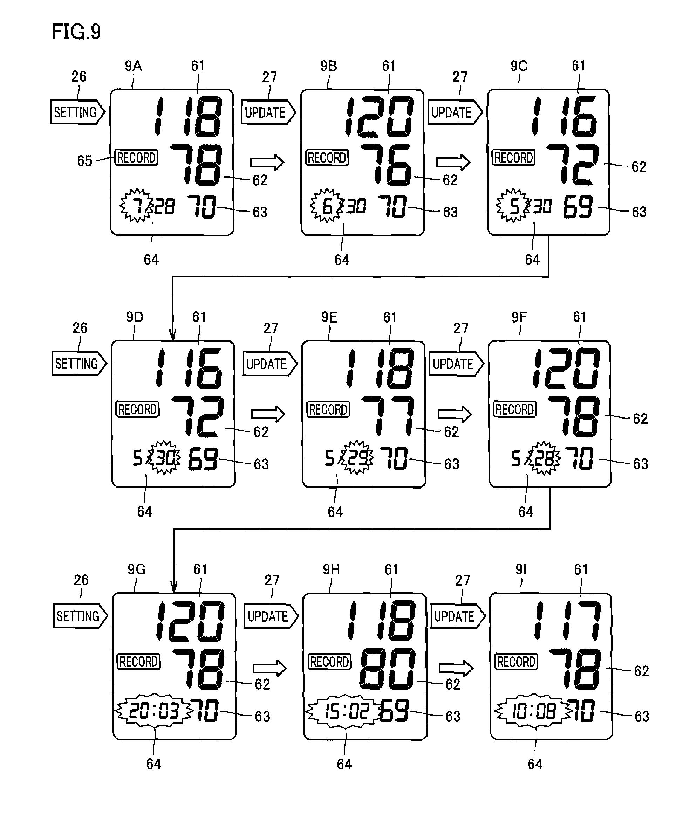

Fig. 9 shows display examples 9A to 9I. In the present second embodiment, examples 9A to 9I are shown on the screen of display unit 2 in response to calling of measurement data from memory 13. While no data is displayed on display unit 2, initially, setting switch 26 is operated once. Then, flag F1 is set to a value indicating the mode of reading measurement data by the month, based on operation signal 253. Memory read unit 232 searches memory 13 based on operation signal 253 provided from storage operation unit 222 and reads record Ri. Specifically, out of record Ri of which data D 1 indicates the latest month, record Ri having time data T indicating the latest time (day, hour(s), minute(s)) is retrieved and read, and provided to display control unit 242. Display control unit 242 causes display unit 2 to display provided record Ri. Here, as to data 64 corresponding to time data T to be displayed, data D1 is displayed in a manner different from others based on the value of flag F1 referred to by display control unit 242. For example, the data blinks.

Thereafter, each time update switch 27 is operated, records R1→R4→R8 are successively called as in the case of operation of month update switch 23 in the first embodiment, and therefore, display on display unit 2 sequentially changes from example 9A → example 9B → example 9C. In example 9B and example 9C, display control unit 242 causes the value indicating data D1, among values indicated by data 64 displayed on display unit 2, to be displayed in a manner different from others, based on operation signal 253 indicating that update switch 27 has been operated while the mode of reading the measurement data by the month is set.

When setting switch 26 is operated again while example 9C is displayed, flag F1 is updated to a value indicating the mode of reading the measurement data by the day, based on operation signal 253. Here, memory read unit 232 searches memory 13 based on the value of flag F1, retrieves and reads record Ri, and provides the record to display control unit 242. Specifically, out of one or more record Ri having data D1 indicating a month the same as the month indicated by data D1 of record R8 that is being displayed on display unit 2, record Ri having data D1 and D2 indicating the most recent date is retrieved and read, and provided to display control unit 242. Display control unit 242 causes display unit 2 to display provided record Ri as shown in example 9D. Here, display control unit 242 causes a value indicated by data D2 of data 64 to be displayed on display unit 2 in a manner different from others, based on the value of flag F1 that is referred to. For example, the value blinks.

Thereafter, each time update switch 27 is operated, records R9→R10 are successively read as in the case of operation of day update switch 24 in the first embodiment, and therefore, display on display unit 2 (example 9D) changes to example 9E → example 9f. While example 9D, example 9E and example 9F are displayed, display control unit 242 makes the display manner of the value indicated by data D2 of data 64 that is being displayed different from others, based on operation signal 253 indicating that update switch 27 has been operated while the mode of reading the measurement data by the day is set.

When setting switch 26 is operated again while example 9F is displayed, flag F1 is updated to a value indicating the mode of reading the measurement data by the hour and the minute within the same day, based on operation signal 253. Here, memory read unit 232 searches memory 13 based on the value of flag F1, retrieves and reads record Ri, and provides the record to display control unit 242. Specifically, out of one or more record Ri having data D1 and D2 indicating the month and day the same as the month and day indicated by data D1 and D2 of record R10 that is being displayed on display unit 2, record Ri having data D3 and D4 indicating the most recent hour(s) and minute(s) is retrieved and read, and provided to display control unit 242. Display control unit 242 causes display unit 2 to display provided record Ri as shown in example 9G. Here, data 64 to be displayed (data 64 corresponding to time data T) shows data D3 and D4. The data is displayed in a manner different from others, based on the value of flag F1 that is referred to by display control unit 242. For example, the data blinks.

Thereafter, each time update switch 27 is operated, records R10→R11→R12 are successively called as in the case of operation of individual switch 25 in the first embodiment, and therefore, example 9G on the screen of display unit 2 changes to example 9H → example 9I. While example 9G, example 9H and example 9I are displayed, display control unit 242 causes the value indicating data D3 and D4 indicated by data 64 to be displayed in a manner different from others, based on operation signal 253 indicating that update switch 27 has been operated while the mode of reading the measurement data by the hour and minute within the same day is set.

Thereafter, when setting switch 26 is operated, flag F 1 is updated to the value indicating the mode of reading the measurement data by the month. Therefore, as described previously, the representative blood pressure measurement data of each month is called.

It is noted that a rotary switch or the like may implement setting switch 26 and update switch 27.

As in the present second embodiment, when setting switch 26 and update switch 27 are provided instead of month update switch 23, day update switch 24 and individual switch 25 in the first embodiment as well, measurement data on a desired date can be called readily and quickly. Namely, instead of retrieving and displaying measurement data stored in memory 13 simply in the descending order from latest data to past data as in a conventional example, representative measurement data of each month, representative measurement data of each day, and measurement data by the hour and minute in one day can be called for each operation of setting switch 26 and update switch 27. Consequently, the data at a desired measurement time out of a large amount of measurement data stored in memory 13 can be called readily and quickly.

### (Third Embodiment)

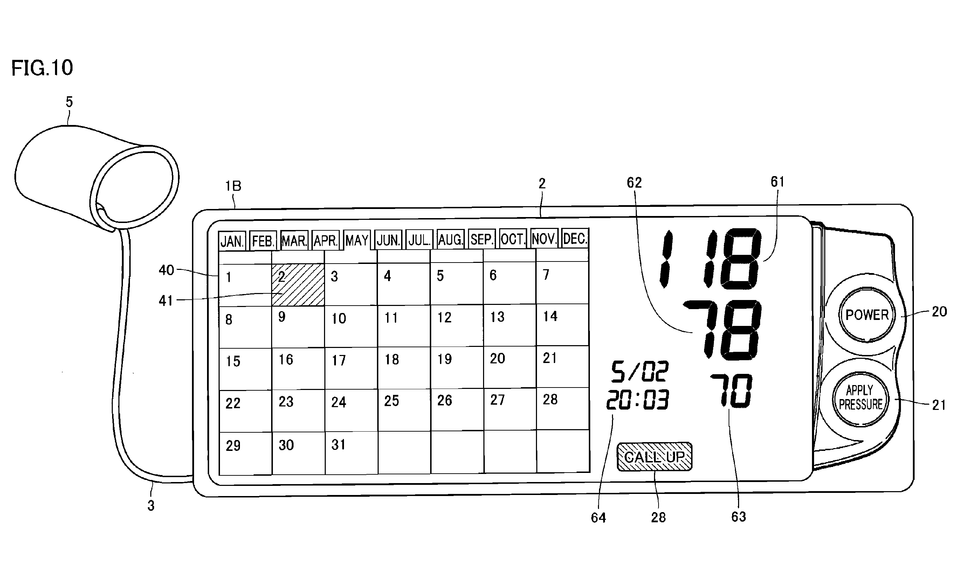

Fig. 10 shows appearance of an electronic blood pressure monitor 1B according to a third embodiment. Electronic blood pressure monitor 1B according to the present embodiment includes power switch 20 and measurement switch 21 as operation unit 4, as well as display unit 2 capable of displaying a calendar 40 and measurement data (data of record Ri) thereon.

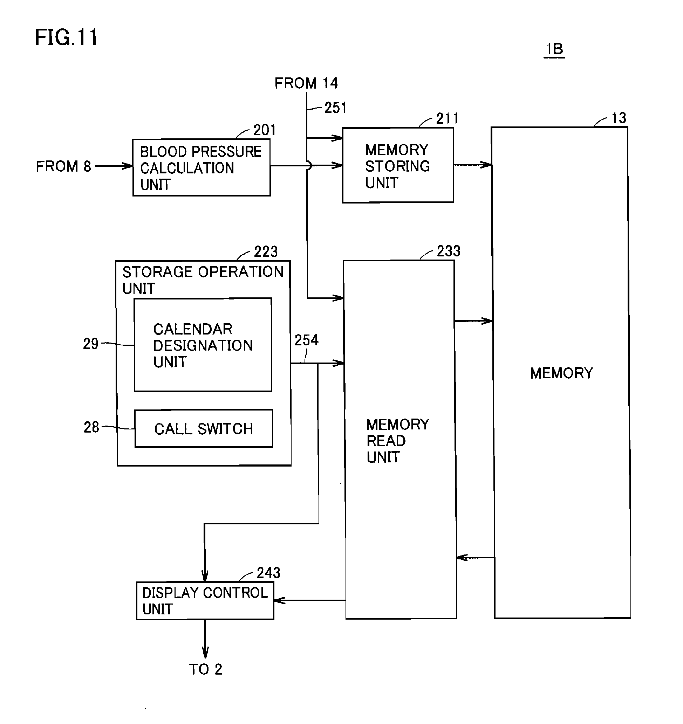

Fig. 11 shows a functional configuration of electronic blood pressure monitor 1A according to the present third embodiment. The functional configuration here is different from the functional configuration in Fig. 4 in that a storage operation unit 223 outputting an operation signal 254, a memory read unit 233, and a display control unit 243 are provided instead of storage operation unit 221, memory read unit 231, and display control unit 241, respectively. Other configurations are the same as in Fig. 4.

When the subject performs an operation for designating desired month and day in calendar 40 displayed as shown in Fig. 10, operation signal 254 indicating the designated month and day is provided to memory read unit 233. Then, memory read unit 233 retrieves and reads from memory 13 one or more record Ri having time data T indicating the month and day indicated by operation signal 254, and outputs the record to display control unit 243. Therefore, display control unit 243 causes display unit 2 to display the measurement data of read record Ri. Specifically, in the screen the same as the screen displaying calendar 40, data 64 showing month, day, hour(s), and minute(s) of time data T of read record Ri, data 61 corresponding to data SBP, data 62 corresponding to data DBP, and data 63 corresponding to data PLS are displayed.

The month and day in calendar 40 can be designated, for example, by touching display unit 2 implemented by a touch screen with a finger or the like. Alternatively, a cross-shaped switch, a rotary switch, or the like may be provided in operation unit 4, and desired month and day may be designated by operating such a switch to move a cursor 41 or the like over calendar 40.

Storage operation unit 223 includes a call switch 28 and a calendar designation unit 29. Call switch 28 and calendar designation unit 29 are configured integrally with display unit 2. While display control unit 243 causes display unit 2 to display calendar 40, the subject designates a desired date on calendar 40 (designation using calendar designation unit 29) by operating the touch screen described previously, and operates call switch 28. Here, operation signal 254 indicating the operation detail, that is, indicating the designated date, is provided to memory read unit 233. Memory read unit 233 reads record Ri by searching memory 13, based on provided operation signal 254. Specifically, out of one or more record Ri storing time data T indicating the date the same as the date indicated by operation signal 254, record Ri storing data T indicating the latest (most recent) time is retrieved and read, and provided to display control unit 243. Display control unit 243 causes display unit 2 to display data as shown in Fig. 10, based on provided record Ri.

Though the month and day is designated by using calendar 40, only the month may be designated using the calendar and measurement data representative of the designated month may be called.

Data for displaying calendar 40 may be created by CPU 16, or may be stored in advance in a prescribed storage area of memory 13 and read and displayed on display unit 2.

In each embodiment, if a plurality of pieces of blood pressure measurement data (data SBP, data DBP, data PLS) associated with time data T corresponding to the time (month and day) indicated by operation signals 252, 253 and 254 are stored, that is, if a plurality of records Ri having corresponding time data T are stored, the blood pressure measurement data associated with time data T indicating the most recent time is retrieved and read as representative data. Namely, the measurement data stored in memory 13 most recently or finally is retrieved and read as the data representative of the plurality of pieces of blood pressure measurement data. The representative data, however, is not limited to the most recent measurement data. For example, measurement data (record Ri) having a highest value of data SBP or measurement data (record Ri) indicating a median value of data SBP may serve as representative data.

It is noted that the configuration of memory 13 is not limited to that shown in Fig. 3. For example, memory 13 is provided with a storage area separately for each month. Record Ri storing data of a blood pressure value, a pulse rate, and day and time may be recorded at the time of measurement in such an area provided separately. Then, each time determination that the month has been updated is made based on time keeping data 251, a memory area for storing measurement data of a next month is secured from the beginning of an empty area of memory 13. Thus, since an area for storing measurement data is secured as necessary, a large amount of measurement data can be recorded efficiently with a limited memory capacity.

According to each embodiment described above, among a large amount of blood pressure measurement data (record Ri) stored in memory 13, measurement data at a specific (desired) measurement time can be called quickly with a simple operation, without a complicated operation or repeated operations many times, and therefore, operability for managing blood pressure is excellent.

It should be understood that the embodiments disclosed herein are illustrative and non-restrictive in every respect. The scope of the present invention is defined by the terms of the claims, rather than the description above, and is intended to include any modifications within the scope and meaning equivalent to the terms of the claims.

## Claims

1. An electronic blood pressure monitor, comprising:
a blood pressure measurement unit having a cuff (5) attached to a blood pressure measurement site, a pressurization and depressurization unit (19, 20) adjusting a pressure to be applied to said cuff, a pressure detection unit (7) detecting a pressure in said cuff adjusted by said pressurization and depressurization unit, and a blood pressure calculation unit (201) calculating a blood pressure based on the pressure detected by said pressure detection unit;
a memory (13);
a display unit (2);
a storing unit (211) storing in said memory, data of the blood pressure calculated by said blood pressure calculation unit in association with measurement time data indicating a time when blood pressure of the blood pressure data was measured;
a storage operation unit (221) operated for designation of a desired time;
a reading unit (231) retrieving and reading said blood pressure data associated with said measurement time data indicating said desired time from said memory, based on operation detail of said storage operation unit; and
a display control unit (241) causing said display unit to display said blood pressure data read by said reading unit.

2. The electronic blood pressure monitor according to claim 1, wherein
said measurement time data includes data indicating a month when measurement was conducted, and
said storage operation unit includes a month operation unit for successively retrieving and reading representative said blood pressure data out of one or more piece of said blood pressure data associated with said measurement time data indicating each month, each time it is operated.

3. The electronic blood pressure monitor according to claim 1, wherein
said measurement time data includes data indicating a month and a day when measurement was conducted, and
said storage operation unit includes a day operation unit for successively retrieving and reading representative said blood pressure data out of one or more piece of said blood pressure data associated with said measurement time data indicating each day in a prescribed month, each time it is operated.

4. The electronic blood pressure monitor according to claim 1, wherein
said storage operation unit includes an individual operation unit for successively retrieving and reading one or more piece of said blood pressure data associated with said measurement time data indicating prescribed month and day, each time it is operated.

5. The electronic blood pressure monitor according to claim 1, wherein
said measurement time data includes data indicating a month, a day, and hour and minute when measurement was conducted, and
each time said storage operation unit is operated, said storage operation unit selectively sets said reading unit to any operating mode of
a month operation mode for retrieving and reading representative said blood pressure data out of one or more piece of said blood pressure data associated with said measurement time data indicating each month,
a day operation mode for successively retrieving and reading representative said blood pressure data out of one or more piece of said blood pressure data associated with said measurement time data indicating each day in a prescribed month, each time it is operated, and
an individual operation mode for retrieving and reading one or more piece of said blood pressure data associated with said measurement time data indicating prescribed month and day, each time it is operated.

6. The electronic blood pressure monitor according to claim 5, wherein
said display control unit further causes said display unit to display, in a prescribed manner, data indicating said month, of said measurement time data associated with said representative blood pressure data, when the operating mode of said reading unit is set to said month operation mode.

7. The electronic blood pressure monitor according to claim 5, wherein
said display control unit further causes said display unit to display, in a prescribed manner, data indicating said day in the prescribed month, of said measurement time data associated with said representative blood pressure data, when the operating mode of said reading unit is set to said day operation mode.

8. The electronic blood pressure monitor according to claim 5, wherein
said measurement time data includes data indicating a month, a day, and hour and minute when measurement was conducted, and
said display control unit further causes said display unit to display, in a prescribed manner, data indicating said hour and minute, of said measurement time data associated with retrieved and read said blood pressure data, when the operating mode of said reading unit is set to said individual operation mode.

9. The electronic blood pressure monitor according to claim 5, further comprising a timer keeping time, wherein
said representative blood pressure data is **characterized in that** associated said measurement time data indicates a time closest to a current time kept by said timer.

10. The electronic blood pressure monitor according to claim 1, wherein
said storage operation unit includes a calendar display unit displaying a calendar on a screen for designation of said desired time, and retrieves and reads representative said blood pressure data out of one or more piece of said blood pressure data associated with said measurement time data indicating a month designated in said calendar displayed on said calendar display unit.

11. The electronic blood pressure monitor according to claim 10, further comprising a timer keeping time, wherein
said representative blood pressure data is **characterized in that** associated said measurement time data indicates a time closest to a current time kept by said timer.

12. The electronic blood pressure monitor according to claim 1, wherein
said storage operation unit includes a calendar display unit displaying a calendar on a screen for designation of said desired time, and retrieves and reads representative said blood pressure data out of one or more piece of said blood pressure data associated with said measurement time data indicating a month and a day designated in said calendar displayed on said calendar display unit.

13. A data display method in an electronic blood pressure monitor including a blood pressure measurement unit having a cuff (5) attached to a blood pressure measurement site, a pressurization and depressurization unit (9, 10) adjusting a pressure to be applied to said cuff, a pressure detection unit (7) detecting a pressure in said cuff adjusted by said pressurization and depressurization unit, and a blood pressure calculation unit (201) calculating a blood pressure based on the pressure detected by said pressure detection unit, a memory (13), a display unit (2), and a storing unit (211) storing in said memory, data of the blood pressure calculated by said blood pressure calculation unit in association with measurement time data indicating a time when blood pressure of the blood pressure data was measured, comprising:
a storage operation step of performing an operation for designating a desired time;
a reading step of retrieving and reading said blood pressure data associated with said measurement time data indicating said desired time from said memory, based on operation detail by said storage operation step; and
a display step of displaying said blood pressure data read in said reading step on said display unit.
